(19) Europäisches Patentamt
European Patent Office
Office européen des brevets

(11) Veröffentlichungsnummer: **0 013 026**
**A1**

(12) # EUROPÄISCHE PATENTANMELDUNG

(21) Anmeldenummer: 79105367.1

(22) Anmeldetag: 24.12.79

(51) Int. Cl.³: C 07 C 41/03
C 07 C 43/13

(30) Priorität: 02.01.79 DE 2900030
26.10.79 DE 2943353

(43) Veröffentlichungstag der Anmeldung:
09.07.80 Patentblatt 80/14

(84) Benannte Vertragsstaaten:
BE CH DE FR GB IT NL

(71) Anmelder: Henkel Kommanditgesellschaft auf Aktien
Postfach 1100 Henkelstrasse 67
D-4000 Düsseldorf 1(DE)

(72) Erfinder: Meffert, Alfred, Dr.
Itterstrasse 33
D-4000 Düsseldorf 13(DE)

(72) Erfinder: Baumann, Horst, Dr.
Vogelwarte 17
D-5672 Leichlingen(DE)

(54) **Verfahren zur Herstellung von Etheralkoholen.**

(57) Zur Herstellung von mono- und polyfunktionellen Etheralkoholen hoher Farbqualität werden Epoxyverbindungen der Formel I,

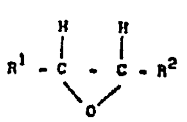

I

in der
a) $R^1$ und $R^2$ Wasserstoff oder $C_1$ - $C_{16}$ -Alkylreste darstellen, wobei $R^1$ + $R^2$ 6 bis 18 Kohlenstoffatome enthalten, oder
b) $R^1$ Wasserstoff und $R^2$ eine Gruppe $R^3$ - O - $CH_2$ - mit mit $R^3$ = $C_6$ - $C_{18}$ -Alkyl darstellt,
mit $C_1$ - $C_{10}$- Alkoholen, die 1 bis 4 OH-Gruppen enthalten, im Molverhältnis 1 : (1,1 - 10) in Abwesenheit von Lösungsmitteln und in Gegenwart von Schwefelsäure oder aromatischen Sulfonsäuren mit höchstens 8 Kohlenstoffatomen bei 50 bis 130°C miteinander umgesetzt. Danach wird die zugesetzte Säure neutralisiert und der nicht umgesetzte Alkohol bei einer Temperatur von höchstens 150°C unter verminderten Druck abdestilliert.

EP 0 013 026 A1

Patentanmeldung
4000 Düsseldorf, den
Henkelstrasse 67

HENKEL KGaA 0013026
ZR-FE/Patente
Dr.Cla/Rs.

P a t e n t a n m el d u n g

EU 5883/6085

"Verfahren zur Herstellung von Etheralkoholen"

---

Die Erfindung betrifft ein Verfahren zur Herstellung von mono- und polyfunktionellen Etheralkohlen hoher Farbqualität.

Etheralkohole, wie sie durch Umsetzung von $C_8$ - bis $C_{20}$-Epoxyalkanen und $C_6$- bis $C_{18}$-Alkylglycidylethern mit mono- oder polyfunktionellen Alkoholen erhalten werden können, sind wertvolle Ausgangsstoffe für die Herstellung von oberflächenaktiven Substanzen. Beispielsweise lassen sich aus diesen Etheralkoholen durch Alkoxylierung oder Sulfatierung Netzmittel, Emulgatoren, Solubilisatoren oder Tenside für Waschmittel herstellen. Geeignete Epoxyalkane sind aus Olefinen durch Epoxidation leicht und billig zugänglich. Geeignete Alkylglycidylether werden aus Epichlorhydrin und Alkalimetallalkoholaten erhalten. Die Umsetzung mit Alkoholen zu Etheralkoholen erfolgt in sauer oder basisch katalysierter Ringöffnungsreaktion.

Aus der US-PS 3 475.499 ist bereits ein Verfahren bekannt, bei dem man längerkettige Epoxyalkane mit Wasser oder Alkohol im Überschuß bei Temperaturen zwischen 20 und 200°C in Gegenwart von Basen wie Alkalimetalloxiden, NaOH, KOH, $Ba(OH)_2$,

/2

$Ca(OH)_2$, $Na_2CO_3$, $K_2CO_3$, $BaCO_3$ und $CaCO_3$ oder in Gegenwart von Säuren wie $H_2SO_4$, $HCl$, $H_3PO_4$, $H_2SO_3$, $HCN$, $H_3BO_3$ und $H_2B_4O_7$ umsetzt. Weitherhin ist aus der US-PS 3 607 778 ein Verfahren bekannt, bei dem die Ringöffnungsreaktion in Gegenwart von Borflouridetherat abläuft.

Es hat sich gezeigt, daß die Reaktion zwischen Epoxyverbindungen und Alkohlen zu mehr oder weniger dunkel gefärbeten Produkten führt, wenn sie in Gegenwart von starken Mineralsäuren und bei Temperaturen abläuft, unter denen eine ausreichend schnelle Umsetzung stattfindet. Für eine Reihe von Einsatzgebieten ist die Anwesenheit von dunkel gefärbten Nebenprodukten nicht störend. Für die Weiterverarbeitung zu Waschmitteltensiden oder Solubilisatoren werden dagegen hellfarbige Produkte verlangt. In diesen Fällen sind aufwendige und umständliche Reinigungsoperationen zur Abtrennung der dunkel gefärbten Nebenprodukte notwendig. Entsprechendes gilt, wenn man das als Alkoxylierungskatalysator bekannte Natriummethylat einsetzt. Verwendet man Lewis-Säuren, wie z.B. Bortrifluorid, als Katalysator, so wird die Bildung von dunkelfarbigen Nebenprodukte zurückgedrängt. Borfluorid zersetzt sich jedoch unter den Reaktionsbedingungen unter Bildung von Fluorwasserstoff, der zu Korrosionserscheinungen an den Anlagenteilen führt, weshalb Bortrifluorid als Katalysator für eine Herstellung im technischen Maßstab nicht in Frage kommt. Entsprechendes gilt auch für die übrigen Lewis-Säuren, die flüchtige Säuren abspalten.

Aus der US-PS 4 079 086 ist bekannt, daß man die Bildung dunkel gefärbter Nebenprodukte vermeiden kann, wenn man die Umsetzung unter Verwendung von Schwefelsäure als

Katalysator in siedendem Benzol, gegebenfalls unter Druck, durchführt. Die Verwendung von inerten Lösungsmitteln als Reaktionsmedium führt aber zu schlechten Raum/Zeit-Ausbeuten.

Der Erfindung lag daher die Aufgabe zugrunde, ein Verfahren zur Herstellung von Etheralkoholen aus Epoxyalkanen oder Alkylglycidylethern und mono- oder polyfunktionellen Alkoholen bereitzustellen, das Etheralkohole mit hoher Farbqualität liefert und in technischem Maßstab anwendbar ist, ohne zu Korrosionserscheinungen an den Anlagenteilen zu führen.

Diese Aufgabe wird erfindungsgemäß gelöst durch ein Verfahren zur Herstellung von Etheralkoholen durch Umsetzen von Epoxyalkanen oder Alkylglycidylethern mit überschüssigen Alkoholen bei erhöhter Temperatur und in Gegenwart von sauren Katalysatoren. Dieses Verfahren ist dadurch gekennzeichnet, daß man Epoxyverbindungen der Formel I,

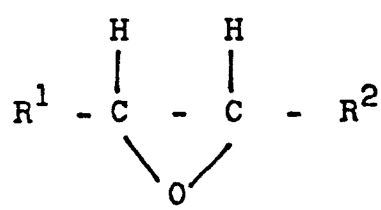

I

in der

a) $R^1$ und $R^2$ Wasserstoff oder Alkylreste mit 1 bis 18 Kohlenstoffatomen darstellen, wobei die Summe der Kohlenstoffatome in den Resten $R^1 + R^2$ 6 bis 18 beträgt, oder

b) $R^1$ Wasserstoff und $R^2$ eine Gruppe der Formel II

$$R^3 - O - CH_2 - \qquad II$$

darstellt, in der $R^3$ ein Alkylrest mit 6 bis 18 Kohlenstoffatomen ist,

mit mono- oder polyfunktionellen Alkoholen, die 1 bis 10 Kohlenstoffatome und 1 bis 4 alkoholische Hydroxylgruppen enthalten, in einem Molverhältnis von 1 : 1,1 bis 1 : 10 in Abwesenheit von Lösungsmitteln und in Gegenwart von Schwefelsäure oder aromatischen Sulfonsäuren mit höchstens 8 Kohlenstoffatomen bei 50 bis 130°C miteinander umsetzt, nach beendeter Umsetzung die zugesetzte Säure neutralisiert und anschließend den nicht umgesetzten Alkohol bei einer Temperatur von höchstens 150°C unter vermindertem Druck abdestilliert.

Epoxyalkane werden aus den entsprechenden Olefinen bzw. Olefingemischen durch Epoxidation nach bekannten Verfahren erhalten. Zu den 1,2-Epoxylalkanen kommt man über 1,2-Monoolefine, die beispielsweise durch Polymerisation von Ethylen mit organischen Aluminiumverbindungen als Katalysatoren oder durch thermisches Cracken von Paraffinkohlenwasserstoffen erhalten werden. Beispiele für bevorzugt verwendbare Epoxyalkane sind die

Verbindungen 1,2-Epoxydodecan, 1,2-Epoxytetradecan, 1,2-Epoxyhexadecan, 1,2-Epoxyoctadecan und Epoxidgemische wie z.B. $C_{12/14}$- 1,2-Epoxid mit ca. 70 Gew.-% $C_{12}$- und ca. 30 Gew.-% $C_{14}$-Epoxyalkan oder $C_{16/18}$-1,2-Epoxid mit ca. 40 Gew.-% $C_{16}$- und ca. 60 Gew.-% $C_{18}$-Epoxyalkan.

Die innenständigen Epoxyalkane erhält man beispielsweise durch Epoxydierung von Olefinen oder Olefingemischen, die durch katalytische Dehydrierung oder durch Chlorierung/ Dehydrochlorierung von linearen Paraffinkohlenwasserstoffen hergestellt werden. Monoolefine mit innenständiger Doppelbindung können auch durch Isomerisierung von $\alpha$-Olefinen hergestellt werden. Bevorzugt eingesetzte innenständige Epoxyalkane aus einer $C_{11/14}$-Olefinfraktion enthalten ca. 22 Gew.-% $C_{11}$-, ca. 30 Gew.-% $C_{12}$-, ca. 26 Gew.-% $C_{13}$-, ca. 22 Gew.-% $C_{14}$-Epoxyalkan. Ein ebenfalls geeignetes Gemisch innenständiger Epoxyalkane aus einer $C_{15/18}$-Olefin-Fraktion enthält ca. 26 Gew.-% $C_{15}$-, ca. 35 Gew.-% $C_{16}$-, ca. 32 Gew.-% $C_{17}$- und ca. 7 Gew.-% $C_{18}$-Epoxyalkan.

Alkylglycidylether können nach bekannten Verfahren, beispielsweise durch Umsetzung von Alkalimetallalkoholaten mit Epichlorlydrin (3-Chlor-1,2-Epoxypropan), enthalten werden. Als geeignete Ausgangsverbindungen sind vor allem die von den geradkettigen aliphatischen $C_6$-$C_{18}$-Alkoholen abgeleiteten Glycidylether zu nennen. Aus dieser homologen Reihe kommen nicht nur die einzelnen Individuen sondern auch entsprechenden Verbindungsgemische zum Einsatz, wie sie aus den technischen Fettalkoholgemischen, z.B. Kokosfettalkohol oder Talgfettalkohol, zugänglich sind.

Geeignete mono- oder polyfunktionellen Alkohle, die 1 bis 10 Kohlenstoffatome und 1 bis 4 alkoholische OH-Gruppen enthalten, sind beispielsweise Methanol, Ethanol, n-Butanol, n-Hexanol, Ethylhexanole, n-Octanol, Ethandiol-1,2, Propandiol-1,2, Glycerin und 1,1,1-Tris-(hydroxymethyl)-propan (Trimethylolpropan).

Ist das Angebot an mono- oder polyfunktionellen Alkoholen im Reaktionsansatz hoch, enthält das Reaktionsprodukt einen hohen Anteil an Umsetzungsprodukten aus einem Mol Epoxyverbindung mit einem Mol Alkohol. Bei geringem Alkoholangebot entstehen in zunehmendem Maße Sekundärumsetzungsprodukte aus einem Mol Epoxyverbindung mit einem Mol bereits gebildetem Etheralkohol. Als besonders vorteilhaft hat sich ein Molverhältnis von etwa 2 bis 6 Mol Alkohol pro Mol Epoxyverbindung erwiesen; hierbei beträgt der Anteil an Primärreaktionsprodukten im Reaktionsgemisch etwa 70 bis über 99 Mol-%.

Die Menge der als Katalysator einzusetzenden starken Säuren hängt in einem gewissen Maße von der Art der Epoxyverbindung und des Alkohols ab. Sie beträgt im allgemeinen 0,05 bis 10 g Säure pro Mol der umzusetzenden Epoxyverbindung; Hiermit wird eine hohe Umsetzungsgeschwindigkeit erzielt.

Als katalytisch wirksame starke Säure, die bei hoher Aktivität unter den Bedingungen des erfindungsgemäßen Verfahrens zu hellfarbigen bis farblosen Produkten führt, ohne nennenswerte Korrosion an den Anlagenteilen zu verursachen, hat sich Schwefelsäure erwiesen, mit der

man eine vollständige Umsetzung innerhalb von etwa 1 bis etwa 5 Stunden durchführen kann. Die Vollständigkeit der Umsetzung kann dabei durch Messung der Epoxid-Zahl oder gaschromatographisch geprüft werden. Ähnliche Ergebnisse werden beim Einsatz von aromatischen Sulfonsäuren mit höchstens 8 Kohlenstoffatomen, beispielsweise Benzolsulfonsäure, Xylolsulfonsäuren und insbesondere p-Toluolsulfonsäure erhalten.

Besonders hellfarbige Umsetzungsprodukte erhält man, wen man die Umsetzung bei Reaktionstemperaturen zwischen 70 und 90°C durchführt.

Das erfindungsgemäße Verfahren gestattet es, Etheralkohole von so guter Farbqualität herzustellen, daß deren Farbe, gemessen im Lovibond-Colorimeter in einer 4"-Küvette einen Gelbwert von maximal etwa 5 hat, während der Rot- und der Blauwert praktisch gleich Null sind.

Nach beendeter Umsetzung wird zunächst die als Katalysator eingesetzte Säure neutralisiert. Hierzu können grundsätzlich alle starken anorganischen Basen, z. B. NaOH, KOH, LiOH oder organischen Basen z.B. Alkalialkoholat oder quartäre Ammoniumbasen verwendet werden. Besonders geeignet ist dabei Natriummethylat.Überschüssigen Alkohol trennt man nach der Neutralisation der Säure durch Destillation unter vermindertem Druck ab. Hierbei ist eine Destillationstemperatur von höchstens 150°C einzuhalten; bei höheren Temperaturen erfolgt eine Verfärbung der Umsetzungsprodukte. Je nach der Dauer der Temperatureinwirkung auf das Reaktionsgemisch kann es zur Herstellung hellfarbiger Produkte

zweckmäßig sein, durch Verringerung des Destillationsdruckes auch bei niedrigeren Destillationstemperaturen als 150°C den Alkohol abzutrennen.

Die erfindungsgemäß hergestellten Umsetzungsprodukte sind farblos bis schwach gelblich gefärbt und können direkt weiterverarbeitet werden, während die Produkte, die nach den Verfahren des Standes der Technik hergestellt werden, mehr oder weniger dunkelfarbig sind und je nach Verwendungszweck gereinigt oder gebleicht werden müssen.

/9

## Beispiele

### Beispiel 1

36,6 kg. Ethandiol-1,2 (590 Mol) wurden mit 0,03 kg konzentrierter Schwefelsäure als Katalysator in einem beheizbaren Rührkessel von 0,115 $m^3$ Inhalt auf 73°C ± 3°C erhitzt. Man ließ dann unter Rühren innerhalb von ca. 0,5 Stunden 63,4 kg (300 Mol) eines technisch hergestellten innenständigen $C_{11}$-$C_{14}$-Epoxyalkangemisches mit einer Kettenlängenverteilung von ca. 22 Gew.-% $C_{11}$-, ca. 30 Gew.-% $C_{12}$-, ca. 26 Gew.-% $C_{13}$- und ca. 22 Gew.-% $C_{14}$-Epoxyalkane zulaufen. Nachdem die Reaktion angesprungen war, was an einer Temperaturerhöhung des Reaktionsgemisches erkennbar war, richtete man den weiteren Zulauf so ein, daß die Temperatur innerhalb von 78 ± 3°C konstant gehalten wurde. Diese Temperatur wurde nach der Zugabe des Epoxyalkans in das Reaktionsgefäß noch weitere 2,5 Stunden aufrechterhalten, wobei die Epoxid-Zahl ständig zurückging. Als nach ingesamt 3 Stunden kein Epoxid mehr nachgewiesen werden konnte, wurde die zugesetzte Schwefelsäure in der Hitze mit einer äquivalenten Menge einer 30 %igen Lösung von Nariummethylat in Methanol neutralisiert. Anschließend wurde das überschüssige Ethandiol-1,2 bei 5 Torr und 90 bis 130°C abdestilliert. Als Rückstand verblieben 75 kg Reaktionsprodukt, das zu 86,2 Gew.-% aus dem Primärumsetzungsprodukt des eingesetzten Epoxyalkans mit Ethandiol-1,2 und zu 13,8 Gew.% aus dem Umsetzungsprodukt des eingesetzten Epoxyalkans mit dem Primärumsetzungsprodukt bestand.

Die Farbe des Umsetzungsprodukts, gemessen im Lovibond-Colorimeter in 4"-Küvette ließ sich durch folgende Parameter beschreiben:
G = 1,0, R = 0, B = 0.

Beispiele 2 bis 16

In grundsätzlich gleicher Weise wurden weitere Umsetzungsprodukte aus Epoxyalkanen und Alkoholen hergestellt. Als Ausgangsmaterialien wurden neben dem in Beispiel 1 beschriebenen innenständigen $C_{11}$- $C_{14}$-Epoxyalkangemisch ("i-11/14") technische 1,2-Epoxyalkangemische der Kettenlänge $C_{12}$ - $C_{14}$ ("α - 12/14-Epoxid") und der Kettenlänge $C_{16}$ - $C_{18}$ ("α - 16/18-Epoxid") eingesetzt. Ausgangsprodukte, Reaktionsbedingungen und Farbwerte der Reaktionsprodukte sind in der folgenden Tabelle 1 wiedergegeben.

/11

Tabelle I

| Beispiel | Epoxid | Alkohol | Molverhältnis Alkohol/Epoxid | $H_2SO_4$ conc. (g/Mol Epoxid) | Rk.-temp. (°C) | Rk.-zeit (h) | Farbe (Lovibond, 4"-Küvette) G | R | B |
|---|---|---|---|---|---|---|---|---|---|
| 2 | i-11/14 | Glycerin | 5:1 | 0,5 | 90 | 2 | 3,0 | 0 | 0 |
| 3 | i-11/14 | TMP *) | 3:1 | 0,1 | 90 | 1 | 2,9 | 0 | 0 |
| 4 | i-11/14 | n-Octanol | 2:1 | 2,0 | 80 | 3 | 2,0 | 0 | 0 |
| 5 | i-11/14 | Methanol | 6:1 | 0,1 | reflux | 3 | 2,0 | 0 | 0 |
| 6 | α-12/14 | Ethandiol-1,2 | 3:1 | 0,1 | 78 | 3 | 0,4 | 0 | 0 |
| 7 | α-12/14 | Glycerin | 3:1 | 0,3 | 90 | 3 | 0,9 | 0 | 0 |
| 8 | α-12/14 | Propandiol-1,2 | 2:1 | 0,7 | 75 | 3 | 0 | 0 | 0 |
| 9 | α-12/14 | TMP *) | 5:1 | 0,3 | 90 | 1 | 2,5 | 0 | 0 |
| 10 | α-12/14 | Methanol | 6:1 | 2,0 | reflux | 3 | 0 | 0 | 0 |
| 11 | α-16/18 | Ethandiol-1,2 | 3:1 | 0,5 | 78 | 3 | 0,9 | 0 | 0 |
| 12 | α-16/18 | Propandiol-1,2 | 2:1 | 1,0 | 80 | 3 | 0 | 0 | 0 |
| 13 | α-16/18 | Glycerin | 2:1 | 2,0 | 90 | 3 | 0 | 0 | 0 |
| 14 | α-16/18 | TMP *) | 2:1 | 0,1 | 90 | 4 | 0,2 | 0 | 0 |
| 15 | α-16/18 | Methanol | 6:1 | 0,5 | reflux | 3 | 0 | 0 | 0 |
| 16 | α-16/18 | n-Octanol | 2:1 | 5,0 | 90 | 6 | 0 | 0 | 0 |

*) = 1,1,1-Tris(hydroxymethyl)-propan, Trimethylolpropan

Beispiel 17

186 g Ethandiol-1,2 (3 Mol) und 0,3 g konzentrierte Schwefelsäure wurden in einem 1l-Dreihalskolben mit Rührer, Tropftrichter und Innenthermometer auf 90°C erhitzt. Man ließ dann unter Rühren im Verlauf von ca. 0,5 Stunden 242 g Dodecylglycidylether (1 Mol) zutropfen. Nach beendeter Zugabe wurde das Reaktionsgemisch 1,5 Stunden lang bei 90°C weitergerührt, bis kein Epoxid mehr nachgewiesen werden konnte. Die zugesetzte Schwefelsäure wurde in der Hitze mit einer äquivalenten Menge Natriummethylat in Methanol neutralisiert. Anschließend wurde das überschüssige Ethandiol-1,2 bis 5 Torr und 90 bis 130°C abdestilliert. Es verblieben 300 g Reaktsprodukt, das zu 96,8 Gew.-% aus dem Primärumsetzungsprodukt des eingesetzten Dodecylglycidylethers mit Ethandiol-1,2 und zu 3,2 Gew.-% aus dem Umsetzungsprodukt des eingesetzten Glycidylethers mit dem Primärumsetzungsprodukt bestand.

Die Farbe des Umsetzungsproduktes, gemessen im Lovibond-Colorimeter in 4" - Küvette, ließ sich durch folgende Parameter beschreiben:
G = 0, R = 0, B = 0.

Beispiele 18 bis 24

In Analogie zu Beispiel 17 wurden weitere Umsetzungsprodukte aus Alkylglycidylethern und Alkoholen hergestellt. Als Ausgangsmaterialien wurde neben dem im Beispiel 17 erwähnten Dodecylglycidylether ein technischer Hexadecylglycidylether eingesetzt. Ausgangsprodukte, Reaktionsbedingungen und Farbwerte der Reaktionsprodukte sind in der folgenden Tabelle 2 wiedergegeben.

Tabelle II

| Beispiel | Alkyl-glycidyl-ether | Alkohol | Molverhältnis Alkohol/Epoxid | $H_2SO_4$ conc. (g/Mol Epoxid) | Reaktionstemp. ($C^o$) | Reaktionszeit (h) | Farbe (Lovibond 4"-Küvette) G | R | B |
|---|---|---|---|---|---|---|---|---|---|
| 18 | $C_{12}H_{25}$ | Methanol | 6 : 1 | 1,1 | reflux | 3 | 0 | 0 | 0 |
| 19 | $C_{12}H_{25}$ | Glycerin | 3 : 1 | 1,2 | 90 | 3 | 0,2 | 0 | 0 |
| 20 | $C_{12}H_{25}$ | TMP+) | 3 : 1 | 0,2 | 90 | 3 | 3,0 | 0,2 | 0 |
| 21 | $C_{16}H_{33}$ | Methanol | 6 : 1 | 2,0 | reflux | 2 | 0 | 0 | 0 |
| 22 | $C_{16}H_{33}$ | Ethandiol | 3 : 1 | 1,4 | 80 | 3 | 1,0 | 0 | 0 |
| 23 | $C_{16}H_{33}$ | Glycerin | 3 : 1 | 1,5 | 90 | 3 | 1,0 | 0 | 0 |
| 24 | $C_{16}H_{33}$ | TMP+) | 3 : 1 | 0,4 | 90 | 3 | 2,0 | 0,2 | 0 |

+) TMP = 1,1,1-Tris(hydroxymethyl)-propan, Trimethylolpropan

Beispiel 25

186 g Ethandiol-1,2 (3 Mol) und 1,6 g p-Toluolsulfonsäure werden in einem 1 l- Dreihalskolben mit Rührer, Tropftrichter und Innenthermometer auf 80°C erhitzt. Anschließend wurden unter Rühren im Verlauf von ca. 0,5 Stunden 211 g des in Beispiel 1 beschriebenen technischen Gemisches aus $C_{11}$-$C_{14}$- Epoxyalkanen mit innenständigen Epoxygruppierungen zugetropft. Nach dem Ende der Zugabe wurde das Reaktionsgemisch 2,5 Stunden lang bei 80°C weitergerührt, bis kein Epoxid mehr nachgewiesen werden konnte. Die zugesetzte p-Toluolsulfonsäure wurde in der Hitze mit einer äquivalenten Menge Natriummethylat neutralisiert. Danach wurde das überschüssige Ethandiol-1,2 bis 5 Torr und 90 bis 130°C abdestilliert. Es verblieben 247 g Reaktionsprodukt, das zu 85,4 Gew.-% aus Primärumsetzungsprodukten des eingesetzten Epoxyalkangemisches mit Ethandiol-1,2 und zu 14,6 Gew.-% aus den Umsetzungsprodukten der eingesetzten Epoxyalkane mit den Primärumsetzungsprodukten bestand.

Die Farbe des Umsetzungsproduktes, gemessen im Lovibond-Colorimeter in einer 4"-Rüvette, ließ sich durch folgende Parameter beschreiben: G=4.0; R=0; B=0.

Beispiel 26

Unter den in Beispiel 25 wiedergegebenen Reaktionsbedingungen würde das in den Beispiel 6 bis 10 verwendete technische Epoxyalkangemisch der Kettenlänge $C_{12}$-$C_{14}$ (" α-C 12/14-Epoxid"; ca. 70 Gew.-% $C_{12}$- und ca. 30 Gew.-% $C_{14}$-Epoxyalkan) mit Ethandiol-1,2 im Molverhältnis 1:3 umgesetzt. Die Farbe des dabei erhaltenden Umsetzungsproduktess, gemessen im Lovibond-Colorimeter in einer 4"-Rüvette, ließ sich durch folgende Parameter beschreiben: G=0,5; R=0; B=0.

"Verfahren zur Herstellung von Ehteralkohlen"

Patentansprüche

1. Verfahren zur Herstellung von Etheralkohlen durch Umsetzen von Epoxyalkanen oder Alkylglycidylethern mit überschüssigen Akoholen bei erhöhter Temperatur und in Gegenwart von sauren Katalysatoren, dadurch gekennzeichnet, daß man Epoxyverbindungen der Formel I,

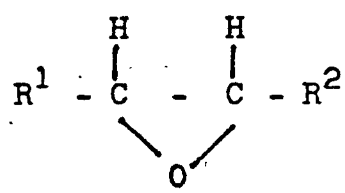

I

in der

a) $R^1$ und $R^2$ Wasserstoff oder Alkylreste mit 1 bis 18 Kohlenstoffatomen darstellen, wobei die Summe der Kohlenstoffatome in den Resten $R^1 + R^2$ 6 bis 18 beträgt, oder

b) $R^1$ Wasserstoff und $R^2$ eine Gruppe der Formel II

$$R^3 - O - CH_2 - \qquad II$$

darstellt, in der $R^3$ ein Alkylrest mit 6 bis 18 Kohlenstoffatomen ist,

mit mono - oder polyfunktionellen Alkoholen, die 1 bis 10 Kohlenstoffatome und 1 bis 4 alkoholische Hydroxylgruppen enthalten, in einem Molverhältnis von 1 : 1,1 bis 1: 10 in Abwesenheit von Lösungsmitteln und in Gegenwart von Schwefelsäure oder aromatischen Sulfonsäuren mit höchstens 8 Kohlenstoffatomen bei 50 bis 13o°C miteinander umsetzt, nach beendeter Umsetzung die zugesetzte Säure neutralisiert und anschließend den nicht umgesetzten Alkohol bei einer Temperatur von höchstens 150°C unter vermindertem Druck abdestilliert.

2. Verfahren nach Anspruch 1, dadurch gekennzeichnet, daß man Epoxyverbindung und Alkohol mit Molverhältnis 1 : 2 bis 1 : 6 miteinander umsetzt.

3. Verfahren nach den Ansprüchen 1 und 2, dadurch gekennzeichnet, daß man die Umsetzung in Gegenwart von 0,05 bis 10 g Säure pro Mol umzusetzendem Epoxyalkan durchführt.

4. Verfahren nach den Ansprüchen 1 : 3, dadurch gekennzeichnet, daß man die Umsetzung in Gegenwart von Schwefelsäure durchführt.

5. Verfahren nach den Ansprüchen 1 bis 3, dadurch gekennzeichnet, daß man die Umsetzung in Gegenwart von p-Toluolsulfonsäure durchführt.

6. Verfahren nach den Ansprüchen 1 bis 5, dadurch gekennzeichnet, daß man die Umsetzung bei 70 bis 90°C durchführt.

7. Verfahren nach den Ansprüchen 1 bis 6, dadurch gekennzeichnet, daß man die zugesetzte Säure mit Natriummethylat neutralisiert.

Europäisches Patentamt

# EUROPÄISCHER RECHERCHENBERICHT

0013026

Nummer der Anmeldung

EP 79 10 5367

| EINSCHLÄGIGE DOKUMENTE | | | KLASSIFIKATION DER ANMELDUNG (Int Cl. 3) |
|---|---|---|---|
| Kategorie | Kennzeichnung des Dokuments mit Angabe, soweit erforderlich, der maßgeblichen Teile | betrifft Anspruch | |
| X | US - A - 2 372 615 (C.A. THOMAS et al.)<br>* Beispiel 1 *<br>-- | 1,4 | C 07 C 41/03<br>43/13 |
| X | US - A - 2 491 533 (D. SWERN)<br>* Beispiele II,IV; Spalte 1, Zeilen 47-48; Spalte 4, Zeilen 6-7 *<br>-- | 1,3-6 | |
| X | US - A - 2 870 220 (C.A. CARTER)<br>* Anspruch 1; Spalte 3, Zeilen 74-75 *<br>-- | 1,4,6,7 | RECHERCHIERTE SACHGEBIETE (Int. Cl. 3)<br>C 07 C 41/03<br>43/13 |
| X | US - A - 2 684 387 (D.P. YOUNG)<br>* Ansprüche 1,4,7 *<br>-- | 1,4,6 | |
| X | US - A - 3 240 819 (VAN R. GAERTNER)<br>* Spalte 3, Zeilen 52-75; Spalte 4, Zeilen 1-12 *<br>---- | 1,3,5,6 | |

KATEGORIE DER GENANNTEN DOKUMENTE

- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

☒ Der vorliegende Recherchenbericht wurde für alle Patentansprüche erstellt.

| Recherchenort | Abschlußdatum der Recherche | Prüfer |
|---|---|---|
| Den Haag | 14-04-1980 | SAGATYS |